(19) Europäisches Patentamt
European Patent Office
Office européen des brevets

(11) Veröffentlichungsnummer: 0 224 849
B1

(12) # EUROPÄISCHE PATENTSCHRIFT

(45) Veröffentlichungstag der Patentschrift: 15.02.89

(51) Int. Cl.⁴: C 07 C 149/415

(21) Anmeldenummer: 86116337.6

(22) Anmeldetag: 25.11.86

(54) **Verfahren zur Herstellung von 4-Mercaptobenzonitrilen und neue 4-Mercaptobenzonitrile.**

(30) Priorität: 05.12.85 DE 3543036

(43) Veröffentlichungstag der Anmeldung: 10.06.87 Patentblatt 87/24

(45) Bekanntmachung des Hinweises auf die Patenterteilung: 15.02.89 Patentblatt 89/7

(84) Benannte Vertragsstaaten: BE CH DE FR GB IT LI

(73) Patentinhaber: BAYER AG, Konzernverwaltung RP Patentabteilung, D-5090 Leverkusen 1 Bayerwerk (DE)

(72) Erfinder: Hagemann, Hermann, Dr., Kandinskystrasse 52, D-5090 Leverkusen 1 (DE)
Erfinder: Sasse, Klaus, Dr., Pützweg 13, D-5060 Bergisch Gladbach 2 (DE)

(56) Entgegenhaltungen:
EP-A-0 193 853
FR-A-2 168 377
FR-A-2 254 562

JUSTUS LIEBIGS ANNALEN DER CHEMIE, Band 716, 1968, Seiten 47-60, Weinheim, Bergstr.; G. BECK et al.: "Nucleophile Substitution an chlorierten Mono- und Dicyan-benzole"
CHEMICAL ABSTRACTS, Band 77, Nr. 15, 9. Oktober 1972, Seite 383, Zusammenfassung Nr. 101077a, Columbus, Ohio, US; Y. TAKIKAWA et al.: "Reactions of organic compounds with sodium hydrosulfide in liquid ammonia. V. Reactions of aromatic halocynides with sodium hydrosulfide in liquid ammonia", & TECHNOL. REP. IWATE UNIV. 1971, 5, 59-65
CHEMICAL ABSTRACTS, Band 104, Nr. 17, 28. April 1986, Seite 661, Zusammenfassung Nr. 148557y,

(56) Entgegenhaltungen: (Fortsetzung)
Columbus, Ohio, US; & JP-A-60 163 857 (HITACHI LTD) 26-08-1985
CHEMICAL ABSTRACTS, Band 77, Nr. 3, 17. Juli 1972, Seite 449, Zusammenfassung Nr. 18874n, Columbus, Ohio, US; Y. TAKIKAWA et al.: "Reactions of organic compounds with sodium hydrosulfide in liquid ammonia. IV. Reactions of aromatic nitriles with sodium hydrosulfide in liquid ammonia", & NIPPON KAGAKUA KAISHI 1972, (4), 766-70

EP 0 224 849 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein Verfahren zur Herstellung von 4-Mercaptobenzonitrilen aus 4-Halogenbenzonitrilen und neue 4-Mercaptobenzonitrile.

Bisher sind lediglich einige 4-Mercaptobenzonitrile bekannt. Unsubstituiertes 4-Mercaptobenzonitril kann hergestellt werden, indem man 4-Aminobenzonitril diazotiert, anschließend mit einer Kaliumxanthogenat-Lösung umsetzt und das Umsetzungsprodukt verseift (siehe DE-A-2 905 992, Ausbeute 46 %). Ferner entsteht es in untergeordneten Mengen bei der Umsetzung von 4-Chlorbenzonitril mit Natriumhydrogensulfid in Gegenwart von flüssigem Ammoniak (siehe Nippon Kagaku Kaishi (4), Seiten 766-770 (1972), Ausbeute 23 %, Hauptprodukt: 4-Chlor-thiobenzamid, weiteres Produkt 4-Mercaptothiobenzamid). Weitere 4-Mercaptobenzonitrile sind in Liebigs Ann. Chem. 716, S. 47 (1968), EP-A-193 853 und C.A. 104, 148557 (1986) beschrieben.

4-Mercaptobenzonitril kann mit bestimmten Cyclohexanderivaten umgesetzt werden. Die dabei entstehenden Produkte sind als nematische flüssig-kristalline Produkte für die Herstellung von Anzeigeelementen geeignet (siehe DE-OS-2 905 992).

Es besteht also ein Bedürfnis nach einem Verfahren, mit dem 4-Mercaptobenzonitrile auf einfache Weise und mit guten Ausbeuten hergestellt werden können.

Es wurde nun ein Verfahren zur Herstellung von 4-Mercapto-benzonitrilen der Formel (I)

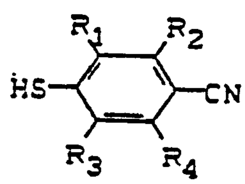

(I),

in der

$R^1$, $R^2$, $R^3$ und $R^4$ unabhängig voneinander jeweils für Wasserstoff, Halogen, Cyanid, gegebenenfalls substituiertes Niederalkyl oder gegebenenfalls substituiertes Niederalkoxy stehen,

gefunden, das dadurch gekennzeichnet ist, daß man 4-Halogenbenzonitrile der Formel (II)

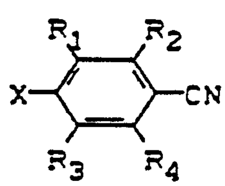

(II),

in der

$R^1$ - $R^4$ die bei Formel (I) angegebene Bedeutung haben und

X für ein Halogenatom steht,

mit Natriumsulfid oder Natriumhydrogensulfid in Gegenwart eines inerten organischen Lösungsmittels umsetzt und anschließend ansäuert.

Soweit in den Formeln (I) und (II) $R_1$, $R_2$, $R_3$ und/oder $R_4$ Halogen bedeuten, kann es sich beispielsweise um Fluor, Chlor oder Brom handeln. Vorzugsweise steht dabei Halogen für Chlor. Soweit in den Formeln (I) und (II) $R_1$, $R_2$, $R_3$ und/oder $R_4$ gegebenenfalls substituiertes Niederalkyl oder Niederalkoxy bedeuten, können darin beispielsweise unsubstituierte oder substituierte $C_1$-$C_6$-Alkylreste vorliegen, wobei als Substiuenten beispielsweise ein oder mehrere Halogenatome, insbesondere Fluoratome, in Frage kommen. Vorzugsweise enthalten gegebenenfalls substituierte Niederalkyl- und Niederalkoxyreste unsubstituierte oder mit 1 bis 3 Fluoratomen substituierte $C_1$-$C_4$-Alkylreste. Besonders bevorzugt sind hier Methyl und Trifluormethyl.

Vorzugsweise stehen in den Formeln (I) und (II) drei der Reste $R_1$, $R_2$, $R_3$ und $R_4$ für Wasserstoff und der vierte dieser Reste für Wasserstoff, Halogen, Cyanid, gegebenenfalls substituiertes Niederalkyl oder gegebenenfalls substituiertes Niederalkoxy.

In Formel (II) steht X vorzugsweise für Fluor, Chlor oder Brom, besonders bevorzugt für Chlor.

Nach dem erfindungsgemäßen Verfahren lassen sich insbesondere 4-Mercaptobenzonitril, 4-Mercapto-5-fluorbenzonitril, 4-Mercapto-5-chlorbenzonitril, 4-Mercapto-5-brombenzonitril, 4-Mercapto-5-methylbenzonitril, 4-Mercapto-5-tri-fluormethylbenzonitril, 2-Chlor-4-mercaptobenzonitril, 2-Trifluormethyl-4-mercaptobenzonitril und 2-Cyano-4-mercaptobenzonitril beispielsweise aus den entsprechenden 4-Chlor-benzonitrilen herstellen.

Die in das erfindungsgemäße Verfahren einzusetzenden Verbindungen der Formel (II) sind entweder bekannt oder lassen sich auf an sich bekannte Weise aus den entsprechenden Carbonsäuren auf einfache Weise

herstellen.

Natriumsulfid kann beispielsweise in reiner Form oder so wie es im Handel erhältlich ist, d. h. kristallwasserhaltig und/oder mit geringen Anteilen von Verunreinigungen (z. B. Polysulfiden und/oder Thiosulfaten), eingesetzt werden. Natriumhydrogensulfid kann ebenfalls in reiner Form oder als technisches Produkt eingesetzt werden. Wenn wasserhaltiges Natriumsulfid oder -hydrogensulfid eingesetzt wird, ist es vorteilhaft, das Wasser vor dem Zufügen der Verbindung der Formel (II) zu entfernen, beispielsweise indem man bei erhöhten Temperaturen ein inertes Gas durch das Gemisch aus Natriumsulfid oder -hydrogensulfid und inertem organischen Lösungsmittel leitet. Pro Mol der Verbindung der Formel (II) kann man beispielsweise 0,8 bis 1,5 Mol Natriumsulfid einsetzen. Vorzugsweise beträgt diese Menge 1,05 bis 1,2 Mol. Pro Mol der Verbindung der Formel (II) kann man beispielsweise 2 bis 4 Mol Natriumhydrogensulfid einsetzen. Vorzugsweise beträgt diese Menge 2,5 bis 3 Mol. Der Einsatz von Natriumsulfid ist bevorzugt.

Für das erfindungsgemäße Verfahren kommen als inerte organische Lösungsmittel beispielsweise N-Methylpyrrolidon, Dimethylformamid, Hexamethylphosphorsäuretriamid, Dimethylacetamid und N-Methylcaprolactam oder andere N,N- disubstituierte offenkettige oder cyclische Carbonsäureamide infrage. Es können auch mehrere Lösungsmittel eingesetzt werden. Bevorzugt ist N-Methylpyrrolidon. Das oder die Lösungsmittel werden vorzugsweise in einer solchen Menge eingesetzt, daß das Reaktionsgemisch gut rührbar ist.

Die erfindungsgemäße Umsetzung kann bei Normaldruck, aber auch vermindertem oder erhöhtem Druck durchgeführt werden. Bevorzugt ist Normaldruck oder leicht erhöhter Druck, z. B. ein solcher im Bereich von 1 bis 5 bar. Geeignete Temperaturen für die erfindungsgemäße Umsetzung sind beispielsweise solche von 90 bis 180° C. Bevorzugt sind solche von 120 bis 160° C.

Die Umsetzung ist im allgemeinen nach 1 bis 10 Stunden beendet. Danach säuert man an, beispielsweise mit wäßriger Mineralsäure, vorzugsweise mit 5 %-iger bis konzentrierter Salzsäure. Die Säuremenge kann dabei beispielsweise so bemessen werden, daß sich nach der Säurezugabe ein pH-Wert von kleiner als 3, vorzugsweise von 0 bis 2 ergibt. Das Ansäuern kann beispielsweise bei Temperaturen im Bereich von -10 bis +50° C durchgeführt werden, bevorzugt sind hier Temperaturen im Bereich von 0 bis 20° C.

Vorzugsweise entfernt man aus dem Reaktionsgemisch der Umsetzung von 4-Halogenbenzonitril mit Natriumsulfid oder -hydrogensulfid das Lösungsmittel (z. B. durch Destillation), nimmt den verbleibenden Rückstand mit Wasser auf und filtriert eventuell dann noch vorliegende Rückstände ab, bevor man mit dem Ansäuern beginnt.

Nach dem Ansäuern, insbesondere wenn dabei eine Temperatur von unter 20°C eingehalten wird, kristallisiert das hergestellte 4-Mercaptobenzonitril der Formel (I) aus und kann beispielsweise durch Zentrifugieren oder Filtrieren abgetrennt werden. Besonders reine Produkte kann man beispielsweise erhalten, indem man das so isolierte 4-Mercaptobenzonitril der Formel (I) mit einem Lösungsmittel aufnimmt, z. B. mit Methylenchlorid, etwas Aktivkohle hinzufügt, diese nach kurzer Verweilzeit abfiltriert, das Filtrat trocknet, z. B. durch Zugabe von Magnesiumsulfat, und anschließend destilliert.

Das erfindungsgemäße Verfahren liefert die 4-Mercaptobenzonitrile der Formel (I) mit guten Ausbeuten. Diese betragen im allgemeinen über 80 % der Theorie. Das ist ein völlig unerwartetes Ergebnis, denn bei der Umsetzung von 4-Chlorbenzonitril mit Natriumhydrogensulfid in Gegenwart von flüssigem Ammoniak anstelle eines inerten organischen Lösungsmittels wird bevorzugt die Nitrilgruppe in eine Thioamidgruppe umgewandelt und nur wenig 4-Mercaptobenzonitril erhalten (siehe Nippon Kagaku Kaishi (4), Seiten 766-770 (1972).

Es wurden auch neue 4-Mercaptobenzonitrile gefunden.

Bei diesen handelt es sich um 4-Mercapto-5-fluorbenzonitril, 4-Mercapto-5-chlorbenzonitril, 4-Mercapto-5-brombenzonitril, 4-Mercapto-5-methylbenzonitril, 4-Mercapto-5-trifluormethylbenzonitril, 2-Chlor-4-mercaptobenzonitril, 2-Methyl-4-mercaptobenzonitril, 2-Trifluormethyl-4-mercaptobenzonitril und 2-Cyano-4-mercaptobenzonitril, also um Verbindungen der Formel (III)

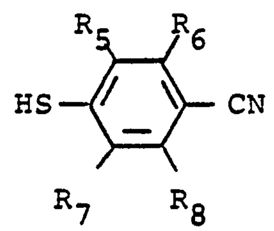

(III)

in der

$R_5$ für Fluor, Chlor, Brom, Methyl oder Trifluormethyl und

$R_6$, $R_7$ und $R_8$ für Wasserstoff oder

$R_6$ für Chlor, Methyl, Trifluormethyl oder Cyano und

$R_5$, $R_7$ und $R_8$ für Wasserstoff stehen.

Die erfindungsgemäßen neuen 4-Mercaptobenzonitrile der Formel (III) können hergestellt werden wie weiter oben beschrieben.

Die neuen 4-Mercaptobenzonitrile können verwendet werden wie das eingangs beschriebene unsubstituierte

4-Mercaptobenzonitril.

Man kann die neuen 4-Mercaptobenzonitrile der Formel (III) aber auch mit Pyri(mi)din-Derivaten der Formel (IV)

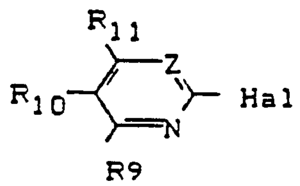

(IV)

in der

$R_9$, $R_{10}$ und $R_{11}$ unabhängig voneinander jeweils für Halogen, Alkyl, gegebenenfalls substituiertes Alkoxy, Halogenalkyl, Alkenyl oder für gegebenenfalls substituiertes Amino stehen, wobei $R_9$ und $R_{10}$ oder $R_{10}$ und $R_{11}$ auch gemeinsam für einen ankondensierter 3-bis 6-gliedrigen carbocyclischen Ring stehen können und wobei mindestens einer der Reste $R_9$, $R_{10}$ und $R_{11}$ für Alkyl oder einen Teil eines 3 bis 6-gliedrigen carbocyclischen Rings stehen,

Z für ein Stickstoffatom oder einer CH-Gruppe steht und

Hal für Chlor oder Brom steht,

in Gegenwart eines Bindemittels für Säuren und gegebenenfalls in Gegenwart eines Verdünnungsmittels bei 30 bis 150°C umsetzten und so Pyri(mi)dyl-thiobenzonitrile der Formel (V)

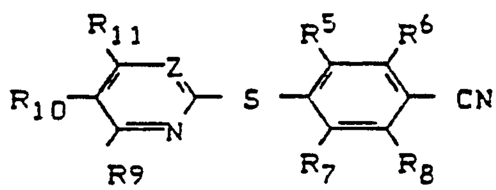

(V)

erhalten, in der $R_5$, $R_6$, $R_7$, $R_8$, $R_9$, $R_{10}$, $R_{11}$ und Z die weiter oben angegeben Bedeutung haben.

Aus den Verbindungen der Formel (V) lassen sich durch Verseifung der CN-Gruppe nach üblichen Methoden die entsprechenden Carbonsäuren herstellen und aus diesen oder deren Metall- oder tert. Ammoniumsalzen in an sich bekannter Weise mit anorganischen Säurehalogeniden, Arylsulfonsäurechloriden oder Chlor-Kohlensäurealkylestern gegebenenfalls in Gegenwart eines Bindemittels für Säuren und/oder eines Verdünnungsmittels, bei 0 bis 100°C Verbindungen der Formel (VI)

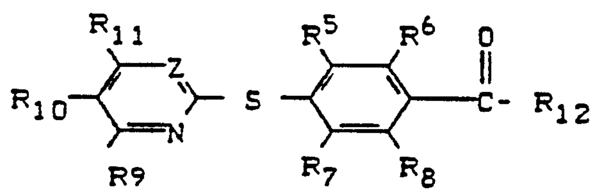

(VI)

in der

$R_5$, $R_6$, $R_7$, $R_8$, $R_9$, $R_{10}$, $R_{11}$ und Z die weiter oben angegebene Bedeutung haben und

$R_{12}$ für Halogen, $-O-SO_2$-Aryl oder den Rest $-O-\overset{O}{\overset{\|}{C}}-O-$Alkyl steht.

Aus den Verbindungen der Formel (VI) lassen sich durch Umsetzung mit Verbindungen der Formel (VII)

$$H-Y-R_{13} \qquad \text{(VII)},$$

in der

Y für Sauerstoff, Schwefel oder einen der Reste

$-\underset{R_{14}}{\underset{|}{N}}-$ oder $-\underset{O-R_{15}}{\underset{|}{N}}-$ steht, wobei $R_{14}$

Wasserstoff oder jeweils gegebenenfalls substituiertes Alkyl oder Alkenyl und $R_{15}$ Wasserstoff oder jeweils gegebenenfalls substituiertes Alkyl oder Alkenyl bedeuten und

$R_{13}$ für gegebenenfalls substituiertes gesättigtes oder ungesättigtes Alkyl steht,

gegebenenfalls in Gegenwart eines Bindemittels für Säuren und/oder eines Verdünnungsmittels Verbindungen der Formel (VIII)

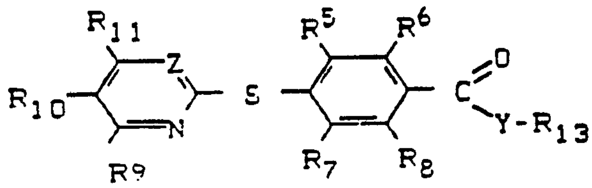

(VIII),

in der

$R_5$, $R_6$, $R_7$, $R_8$, $R_9$, $R_{10}$, $R_{11}$, $R_{13}$, Z und Y die weiter oben angegebene Bedeutung haben, herstellen.

Die Verbindungen der Formel (VIII) besitzen herbizide, insbesondere auch selektiv-herbizide, Eigenschaften und darüber hinaus auch pflanzenwachstumsregulatorische Eigenschaften.

Überraschenderweise zeigen die Verbindungen der Formel (VIII) eine deutlich verbesserte allgemeinherbizide Wirksamkeit gegenüber Schadpflanzen als die aus dem Stand der Technik bekannten Pyridyl- und Pyrimidyl-ether und- thioether, welche chemisch und wirkungsmäßig naheliegende Verbindungen sind. Außerdem zeigen die Verbindungen der Formel (VIII) planzenwachstumsregulatorische Wirksamkeit.

**Beispiele**

**Beispiel 1**

27,7 g (0,21 Mol) Natriumsulfid x $3H_2O$ wurden mit 200 ml N-Methylpyrrolidon gemischt und mit Stickstoff bei 160° C entwässert. Danach wurde auf 140° C abgekühlt und 27,5 g (0,2 Mol) 4-Chlorbenzonitril zugegeben. Nach einer Reaktionszeit von 3 Stunden bei 160° C wurde das N-Methylpyrrolidon abdestilliert, der Rückstand in 250 ml Wasser aufgenommen, filtriert und das Filtrat mit 10 %-iger wäßriger Salzsäure auf einen pH-Wert von 1 eingestellt, wobei durch Kühlung die Temperatur auf weniger 20° C gehalten wurde. Danach wurde abgesaugt, der Filterrückstand in Methylenchlorid aufgenommen, mit Aktivkohle versetzt, erneut filtriert, das Filtrat über Magnesiumsulfat getrocknet, eingeengt und destilliert. Auf diese Weise wurden 23 g (85 % der Theorie) reines 4-Mercaptobenzonitril mit einem Siedepunkt von 88 bis 90° C bei 0,1 mbar und einem Schmelzpunkt von 48 bis 50° C erhalten.

**Beispiele 2 bis 7**

Es wurde verfahren wie in Beispiel 1 beschrieben, jedoch wurden substituierte 4-Chlorbenzonitrile eingesetzt. Die jeweils eingesetzten 4-Chlorbenzonitrile, die isolierten Reaktionsprodukte und diese charakterisierende Daten sind aus Tabelle 1 ersichtlich.

**Tabelle 1**

| Beisp. Nr. | Einsatzprodukt (Formel (II)) | Reaktionsprodukt (Formel (I)) | physikal. Daten |
|---|---|---|---|
| 2 | $X = Cl$; $R_1 = Cl$; $R_2$ bis $R_4 = H$ | $R_1 = Cl$; $R_2$ bis $R_4 = H$ | Smp: 64 - 65° C |
| 3 | $X = Cl$; $R_2 = CH_3$; $R_1$, $R_3$ und $R_4 = H$ | $R_2 = CH_3$; $R_1$ $R_3$ und $R_4 = H$ | Smp: 118 - 120 °c |
| 4 | $X = Cl$; $R_1 = CN$; $R_2$ bis $R_4 = H$ | $R_1 = CN$; $R_2$ bis $R_4 = H$ | Smp: 127° C |
| 5 | $X = Cl$; $R_1 = CF_3$; $R_2$ bis $R_4 = H$ | $R_1 = CF_3$; $R_2$ bis $R_4 = H$ | NMR-Spektrum: 4,0 - 2,22 Quadruplett, 7,3 - 7,8 Multiplett |
| 6 | $X = Cl$; $R_2 = CF_3$ $R_1$, $R_3$ und $R_4 = H$ | $R_2 = CF_3$; $R_1$ $R_3$ ud $R_4 = H$ | Smp: 72 - 74° C |
| 7 | $X = Cl$; $R_1 = F$; $R_2$ bis $R_4 = H$ | $R_1 = F$; $R_2$ bis $R_4 = H$ | NMR-Spektrum: 3,9 - 4,0 Duplett, 7,3 - 7,8 Multiplett. |

**Patentansprüche**

1. Verfahren zur Herstellung von 4-Mercaptobenzonitrilen der Formel (I)

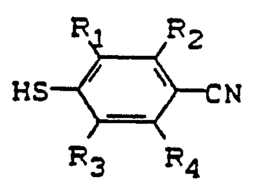

(I),

in der
$R_1$, $R_2$, $R_3$ und $R_4$ unabhängig voneinander jeweils für Wasserstoff, Halogen, Cyanid, gegebenenfalls substituiertes Niederalkyl oder gegebenenfalls substituiertes Niederalkoxy stehen,
dadurch gekennzeichnet, daß man 4-Halogenbenzonitrile der Formel (II)

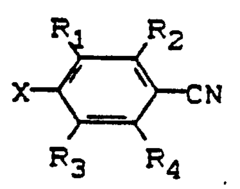

(II),

in der
$R_1$ - $R_4$ die bei Formel (I) angegebene Bedeutung haben und
X für ein Halogenatom steht,
mit Natriumsulfid oder Natriumhydrogensulfid in Gegenwart eines inerten organischen Lösungsmittels umsetzt und anschließend ansäuert.

2. Verfahren nach Anspruch 1, dadurch gekennzeichnet, daß in den Formeln (I) und (II) $R_1$, $R_2$, $R_3$ und/oder $R_4$ für Wasserstoff, Fluor, Chlor, Brom, Cyanid, gegebenenfalls durch Halogenatome substituierte $C_1$-$C_6$-Alkylreste oder gegebenenfalls durch Halogenatome substituierte $C_1$-$C_6$-Alkoxyreste stehen.

3. Verfahren nach Anspruch 1, dadurch gekennzeichnet, daß in den Formeln (I) und (II) drei der Reste $R_1$, $R_2$, $R_3$ und $R_4$ für Wasserstoff und der vierte dieser Reste für Wasserstoff, Halogen, Cyanid, gegebenenfalls substituiertes Niederalkyl oder gegebenenfalls substituiertes Niederalkoxy steht.

4. Verfahren nach Ansprüchen 1 bis 3, dadurch gekennzeichnet, daß man als Lösungsmittel N-Methylpyrrolidon, Dimethylformamid, Hexamethylphosphorsäuretriamid, Dimethylacetamid und/oder N-Methylcaprolactam einsetzt.

5. Verfahren nach Ansprüchen 1 bis 4, dadurch gekennzeichnet, daß man pro Mol der Verbindung der Formel (II) 0,8 bis 1,5 Mol Natriumsulfid oder 2 bis 4 Mol Natriumhydrogensulfid einsetzt.

6. Verfahren nach Ansprüchen 1 bis 5, dadurch gekennzeichnet, daß man mit soviel Säure ansäuert, daß sich ein pH-Wert von kleiner als 3 ergibt.

7. Neue 4-Mercaptobenzonitrile, dadurch gekennzeichnet, daß es sich um 4-Mercapto-5-fluorbenzonitril, 4-Mercapto-5-chlorbenzonitril, 4-Mercapto-5-brombenzonitril, 4-Mercapto-5-methylbenzonitril, 4-Mercapto-5-trifluormethylbenzonitril, 2-Chlor-4-mercaptobenzonitril, 2-Methyl-4-mercaptobenzonitril, 2-Trifluormethyl-4-mercaptobenzonitril oder 2-Cyano-4-mercaptobenzonitril handelt.

## Claims

1. Process for the preparation of 4-mercaptobenzonitriles of the formula (I)

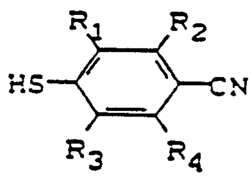

(I)

in which

$R_1$, $R_2$, $R_3$ and $R_4$, independently of one another, in each case represent hydrogen, halogen, cyanide, optionally substituted lower alkyl or optionally substituted lower alkoxy,

characterized in that 4-halogenobenzonitriles of the formula (II)

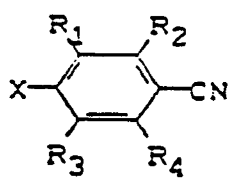

(II)

in which

$R_1$ - $R_4$ have the meaning stated for formula (I) and

X represents a halogen atom,

are reacted with sodium sulphide or sodium hydrogen sulphide in the presence of an inert organic solvent, and are subsequently acidified.

2. Process according to Claim 1, characterized in that $R_1$, $R_2$, $R_3$ and/or R4 in the formulae (I) and (II) represent hydrogen, fluorine, chlorine, bromine, cyanide, $C_1$-$C_6$-alkyl radicals which are optionally substituted by halogen atoms, or $C_1$-$C_6$-alkoxy radicals which are optionally substituted by halogen atoms.

3. Process according to Claim 1, characterised in that three of the radicals $R_1$, $R_2$, $R_3$ and $R_4$ in the formulae (I) and (II) represent hydrogen and the fourth of these radicals represents hydrogen, halogen, cyanide, optionally substituted lower alkyl or optionally substituted lower alkoxy.

4. Process according to Claims 1 to 3, characterized in that N-methylpyrrolidone, dimethylformamide, hexamethylphosphoric triamide, dimethylacetamide and/or N- methylcaprolactam are employed as solvents.

5. Process according to Claims 1 to 4, characterized in that 0.8 to 1.5 mol of sodium sulphide or 2 to 4 mol of sodium hydrogen sulphide are employed per mol of the compound of the formula (II).

6. Process according to Claims 1 to 5, characterized in that the acidification is carried out with sufficient acid to give a pH of less than 3.

7. New 4-mercaptobenzonitriles, characterized in that they are 4-mercapto-5-fluorobenzonitrile, 4-mercapto-5-chlorobenzonitrile, 4-mercapto-5-bromobenzonitrile, 4-mercapto-5-methylbenzonitrile, 4-mercapto-5-trilfuoro-methylbenzonitrile, 2-chloro-4-mercaptobenzonitrile, 2-methyl-4-mercaptobenzonitrile, 2-trifluoromethyl-4-mercaptobenzonitrile or 2-cyano-4-mercaptobenzonitrile.

**Revendications**

1. Procédé de préparation de 4-mercaptobenzonitriles de formule (I)

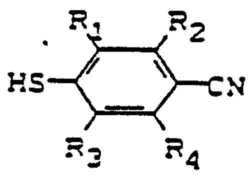

(I),

dans laquelle
$R_1$, $R_2$, $R_3$ et $R_4$ représentent chacun, indépendamment l'un de l'autre, un atome d'hydrogène, un atome d'halogène, un cyanure, un groupe alkyle inférieur éventuellement substitué, ou un groupe alcoxy inférieur éventuellement substitué,
caractérisé en ce qu'on fait réagir des 4-halogéno-benzonitriles de formule (II)

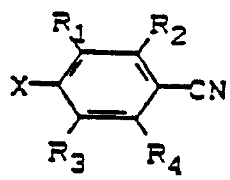

(II),

dans laquelle
$R_1$ - $R_4$ ont les significations indiquées à propos de la formule (I), et
X représente un atome d'halogène,
avec du sulfure de sodium ou de l'hydrogénosulfure de sodium en présence d'un solvant organique inerte, puis on procède à une acidification.

2. Procédé selon la revendication 1, caractérisé en ce que, dans les formules (I) et (II), $R_1$, $R_2$, $R_3$ et /ou $R_4$ représentent chacun un atome d'hydrogène, un atome de fluor, un atome de chlore, un atome de brome, un cyanure, des radicaux alkyle en $C_1$-$C_6$ éventuellement substitués par des atomes d'halogènes ou des radicaux alcoxy en $C_1$-$C_6$ éventuellement substitués par des atomes d'halogènes.

3. Procédé selon la revendication 1, caractérisé en ce que, dans les formules (I) et (II), trois des radicaux $R_1$, $R_2$, $R_3$ et $R_4$ représentent chacun un atome d'hydrogène, tandis que le quatrième de ces radicaux représente un atome d'hydrogène, un atome d'halogène, un cyanure, un groupe alkyle inférieur éventuellement substitué ou un groupe alcoxy inférieur éventuellement substitué.

4. Procédé selon les revendications 1 à 3, caractérisé en ce que, comme solvant, on utilise la N-méthylpyrrolidone, le diméthylformamide, le triamide de l'acide hexaméthylphosphorique, le diméthylacétamide et/ou le N-méthylcaprolactame.

5. Procédé selon les revendications 1 à 4, caractérisé en ce que, par mole du composé de formule (II), on utilise 0,8 à 1,5 mole de sulfure de sodium ou 2 à 4 moles d'hydrogénosulfure de sodium.

6. Procédé selon les revendications 1 à 5, caractérisé en ce qu'on acidifie avec de l'acide en une quantité telle que l'on obtienne un pH inférieur à 3.

7. Nouveaux 4-mercaptobenzonitriles, caractérisés en ce qu'il s'agit du 4-mercapto-5-fluorobenzonitrile, du 4-mercapto-5-chlorobenzonitrile, du 4-mercapto-5-bromobenzonitrile, du 4-mercapto-5-méthylbenzonitrile, du 4-mercapto-5-trifluorométhylbenzonitrile, du 2-chloro-4-mercaptobenzonitrile, du 2-méthyl-4-mercaptobenzonitrile, du 2-trifluorométhyl-4-mercaptobenzonitrile ou du 2-cyano-4-mercaptobenzonitrile.